# DEMANDE DE BREVET EUROPEEN

(11) **EP 1 669 427 A1**
(43) Date de publication de la demande: **14.06.2006**
(21) Numéro de dépôt: 05292556.7
(22) Date de dépôt: 02.12.2005
(51) Int. Cl.: C09J 103/04, C08L 3/04, A61K 8/73, A23L 1/0522

(54) **Nouvelles compositions adhésives aqueuses contenant un mélange comprenant au moins un amidon de blé réticulé à basse température**

(30) Priorité: 09.12.2004 FR 0413137
(71) Demandeur: Roquette Frères, 62136 Lestrem (FR)
(72) Inventeur: Gombert, Hervé, 62232 Hinges (FR); Petitjean, Carole, 59520 Marquette lez Lille (FR); Ingret, Maxime, 62990 Maresquel Ecquemicourt (FR)
(74) Mandataire: Pöpping, Barbara

(57) **Abrégé**

L'invention a pour objet une composition adhésive comprenant un mélange d'au moins un amidon de blé moyennement ou fortement réticulé par l'action d'un trimétaphosphate à basse température, et d'au moins un amidon (plus) faiblement réticulé, de préférence, à basse température.

L'invention a encore pour objet le procédé de réticulation moyenne ou forte, à basse température, par au moins un trimétaphosphate.

L'invention a également pour objet le procédé de mise en oeuvre de la composition selon l'invention comprenant son utilisation pour le collage de cartons ondulés.

L'invention a encore pour objet les cartons ondulés assemblés par la mise en oeuvre d'au moins une composition selon l'invention.

Elle a encore pour objet le produit de mélange qui sont issus d'amidons réticulés par le trimétaphosphate à basse température à au moins deux niveaux différents de réticulation, aptes à être utilisés dans la composition selon l'invention.

Elle vise enfin l'utilisation des nouveaux produits de mélange.

## Description

La présente invention concerne une nouvelle composition adhésive aqueuse, contenant un mélange d'amidons réticulés comprenant au moins un amidon de blé moyennement ou fortement réticulé à basse température et au moins un amidon faiblement réticulé à basse température.

Elle concerne plus particulièrement une nouvelle composition adhésive aqueuse, à base d'un mélange d'amidons réticulés comprenant au moins un amidon de blé moyennement ou fortement réticulé à basse température, conçue pour l'assemblage par collage en vue d'obtenir des produits de carton ondulé.

Elle concerne plus particulièrement un mélange d'amidons réticulés comprenant au moins un amidon de blé moyennement ou fortement réticulé à une température suffisamment basse pour le rendre particulièrement apte à participer à l'élaboration d'une composition adhésive selon l'invention, et au moins un autre amidon plus faiblement réticulé.

Elle concerne encore, et plus précisément, un mélange d'au moins un amidon de blé moyennement ou fortement réticulé à basse température et d'au moins un amidon faiblement réticulé, de préférence, à basse température, éventuellement modifiés en sus avant, pendant ou après l'opération de réticulation, par tout moyen autre que la réticulation.

Elle vise aussi les procédés de fabrication dudit carton ondulé faisant appel à au moins une composition selon l'invention, comprenant un mélange d'au moins un amidon de blé moyennement ou fortement réticulé à basse température et d'au moins un amidon plus faiblement réticulé à basse température, lesdits amidons réticulés pouvant, éventuellement, être modifiés en sus, avant, pendant ou après l'opération de réticulation, par tout moyen autre qu'une réticulation.

Elle concerne encore le carton ondulé obtenu par mise en oeuvre, en un stade au moins de la fabrication, d'au moins une composition selon l'invention.

Par «assemblage par collage» au sens de la présente invention, on entend toute opération propre à assembler les différentes parties constitutives d'un carton ondulé, cannelures, couvertures et, éventuellement, entretoises et à les rendre durablement solidaires.

Lesdites opérations de collage sont communément connues de l'homme de l'art sous le nom de collage dit simple face (« SF »), lorsqu'elles consistent en l'assemblage simple d'une cannelure et d'une couverture, et de collage dit double face (« DF »), lorsqu'elles consistent à rassembler deux couvertures et une cannelure.

Au-delà, des cartons, généralement lourds, sont le plus souvent définis par les appellations courantes « double-double » (« DD »), pour les cartons comportant deux cannelures, et « triple cannelure », pour les cartons qui en comprennent trois.

Il en existe de plus complexes encore, tels que, par exemple, les cartons dits « quadri-cannelures ».

Par « compositions adhésives aqueuses » au sens de la présente invention, on entend toute composition adhésive aqueuse, notamment destinée à la confection de cartons ondulés, comportant une partie d'amidon solubilisé, dite support ou primaire, présentant des propriétés suspensives suffisantes, notamment vis-à-vis de l'amidon granulaire, et une partie d'amidon non solubilisé et/ou seulement hydraté, soit se trouvant à l'état de granules insolubles et/ou de granules au moins partiellement gonflés, également appelée partie secondaire.

Par exemple, de telles compositions faisant appel, en partie secondaire, à un amidon à l'état de granules insolubles, sont le plus souvent élaborées selon les principes connus de l'homme de l'art sous le nom de procédé « Stein-Hall ».

En vertu des règles qui caractérisent ledit procédé, on met en place au moins un amidon, de l'eau et un agent alcalin. On chauffe le tout, par exemple, modérément en cuve ouverte et à la vapeur vive ou, plus violemment, au moyen d'un cuiseur continu, de façon à obtenir une solution colloïdale alcaline présentant notamment des caractéristiques rhéologiques et de capacité de maintien en suspension de particules insolubles, adaptées.

Cette procédure constitue un mode de préparation de la partie dite « primaire » ou « support ».

Selon d'autres règles du procédé « Stein-Hall », on prépare, à la température d'alimentation de l'eau, une dispersion rassemblant au moins de l'eau et de l'amidon granulaire. Le plus souvent, on leur associe un dérivé du bore, couramment, le borax. Le lait obtenu constitue la partie « secondaire ».

On procède à un mélange soigneux des parties primaire et secondaire selon diverses modalités, par exemple, de procédures continues ou discontinues.

Selon un mode voisin, il est possible d'ajouter successivement, à la partie primaire, eau et amidon granulaire et ensuite, le plus fréquemment, le borax.

Ce procédé « Stein-Hall », le plus ancien et très répandu, permet la préparation de compositions aqueuses adhésives ayant un extrait sec final, paramètre essentiel, compris, dans la pratique courante, entre 20 et plus de 30%.

Un autre procédé, comportant une partie primaire avec amidon solubilisé, préparée par augmentation sensible de la température, et sans agent alcalin, et une partie secondaire avec un amidon sous forme de granules insolubles, est connu sous le nom de procédé « PRISTIM® » (brevet européen EP 0 229 741 au nom de la Demanderesse).

Selon un autre procédé encore, connu sous le nom de procédé « Minocar », la partie primaire est associée à une dispersion d'amidon partiellement gonflé (brevet européen EP 0 038 627).

Enfin, le procédé « No-Carrier » se différencie des précédents de par l'existence d'une phase unique obtenue par le gonflement modéré, en milieu alcalin, de l'ensemble de l'amidon (brevet US 3 355 307).

L'existence parallèle de ces procédés trouve sa justification, entre autres raisons, dans la nécessité d'adapter, notamment en termes de rhéologie, les compositions adhésives aqueuses à l'évolution des machines et à leur rapidité sans cesse croissante.

S'il est établi que les procédés dits « No-Carrier » ou « Minocar » se satisfont, la plupart du temps, et compte tenu du principe de gonflement partiel de l'amidon, de l'utilisation d'un amidon non modifié, la Société Demanderesse a, quant à elle, préconisé dans des circonstances qui le méritaient, l'appel à des amidons modifiés, notamment réticulés.

Les autres procédés, initialement basés sur l'usage d'amidon natif, ont été ensuite déclinés de diverses manières, de façon à répondre à l'évolution, les déclinaisons les plus courantes ayant trait, outre l'appel à des extraits secs élevés, à l'utilisation d'amidons modifiés, le plus souvent chimiquement, notamment par réticulation. Les amidons réticulés, en particulier, ont connu un développement important dans la mise en place de formulations de type « Stein-Hall » ou « PRISTIM® ».

Les possibilités de réticulation de l'amidon ont été évoquées à maintes reprises dans la littérature. C'est ainsi, par exemple, que les plus fréquentes d'entre elles, par voie de réaction avec des composés multifonctionnels sont citées dans « Starch : Chemistry and Technology - Volume I - Fundamental Aspects - pages 481 à 483 (Academic Press 1965) » et reprises plus largement encore dans le volume II - Industrial Aspects
- page 446 (Academic Press 1967). Sont évoquées les actions du chlorure de l'acide cyanurique, de l'épichlorhydrine, des trimétaphosphates, et notamment, du trimétaphosphate de sodium (TMPNa), de la vinylsulfone, du formaldéhyde et autres aldéhydes, des dialdéhydes, des diverses résines urée-formaldéhyde, de l'(hydroxyméthyl)éthylèneurée, des diépoxydes, des 1,3,5-trichloro- et 1,3,5-triacryl-s-triazines, du N,N'-méthylènebisacrylamide, de l'oxychlorure de phosphore et de l'hexaméthylène diisocyanate.

A la suite, dans le volume II, pages 447 à 449, divers domaines d'utilisation des amidons réticulés obtenus par ces moyen, sont énumérés, notamment les domaines alimentaires ou chirurgicaux, ainsi que ceux attachés à l'impression textile, aux boues de forage, aux encres et peintures, aux céramiques, aux liants pour noyaux et charbons, aux piles électriques.

Sont aussi cités, les adhésifs, en particulier, ceux destinés aux industries du carton ondulé ou du papier.

Dans ce contexte, l'intérêt des amidons réticulés est souligné, en toute généralité, tant à l'application que dans les dispositifs de recyclage, du fait de la stabilité des colles obtenues avec de tels dérivés, et de la texture dite courte qu'ils leur confèrent.

Dans le cadre des principes généraux ainsi émis de longue date, le brevet US 2 801 242 a décrit, plus particulièrement et dès 1957, l'utilisation, à des fins de réticulation, du trimétaphosphate de sodium (TMPNa).

L'usage en était, dès cette époque, recommandé à une température suffisamment basse de façon à maintenir l'amidon dans son état granulaire.

Plus précisément, il y est préconisé d'opérer à des températures comprises entre 40 et 50°C.

Les exemples font état, majoritairement, d'une température de 50°C et n'envisagent pas de températures inférieures à 45°C.

Sur les règles particulières qui y sont énoncées, et au bénéfice d'une pratique suffisamment répandue, sont venues s'ajouter certaines variantes comme par exemple celle apportée par la demande de brevet japonais JP 63-251486, qui propose, à des fins d'élaboration de parties primaires de compositions adhésives pour le collage du carton ondulé, de pratiquer la réticulation à 40°C puis de pré-gélatiniser l'amidon ainsi obtenu, de façon à ce que cet amidon modifié et pré-gélatinisé présente le degré de gonflement recherché et considéré comme essentiel dans ledit document.

D'autres documents font état de réactions de réticulation pratiquées avec les trimétaphosphates (TMP).

C'est le cas de la demande de brevet US 4 339 331 qui, à des fins d'usage pour la séparation des minerais par flottation, s'abstient pourtant de toute précision relative à la pratique de la réticulation et ne prend en compte que des données tout à fait générales, de concentration, de temps, de température et de pH, qui appartiennent à la littérature citée précédemment, en ne présentant aucune revendication à ce propos.

C'est aussi le cas du brevet US 5 855 946 et de la demande de brevet internationale WO 99/64508, confortés par l'article « Cross-Linked Resistant Starch : Preparation and Properties », paru dans « Cereal Chemistry », Vol. 79, No6, 2002, p.819-825, qui décrit l'utilisation, dans le cadre de taux d'agents de réticulation élevés, de mélanges de trimétaphosphate(s)(TMP) et de tripolyphosphate(s) (TPP), pour l'obtention de produits destinés à des usages alimentaires ou cosmétiques.

Ces derniers documents ne traitent à aucun moment le problème technique tel qu'il se pose dans le domaine des compositions adhésives, notamment destinées à l'assemblage des cartons ondulés.

Les compositions adhésives utiles audit domaine doivent en effet répondre à des impératifs spécifiques. Notamment, si la réticulation de l'amidon permet, en toute généralité, comme la littérature le souligne, de préparer une partie primaire présentant un excellent pouvoir suspensif, notamment vis-à-vis de l'amidon granulaire et d'obtenir, au-delà, des colles à texture courte, propre à une bonne distribution sur les machines modernes, elle s'accompagne, dès lors qu'elle est pratiquée selon les enseignements de l'art antérieur, d'une perte de cohésion et d'une baisse sensible des propriétés adhésives.

Par ailleurs, la Demanderesse a aussi remarqué que l'usage d'un seul amidon réticulé ou de plusieurs amidons ayant subi une réticulation à un niveau comparable, pour la confection d'une composition adhésive, confère à celle-ci une stabilité au cisaillement insuffisante, notamment en termes de viscosité et de constance de celle-ci.

Elle a encore remarqué que, dans ces conditions, un cisaillement intense pouvait provoquer une évolution sensible de la texture, celle-ci étant notamment appréhendée par la détermination d'un ratio établi entre viscosité apparente (viscosité Brookfield) et viscosité à l'écoulement (viscosité Lory), ces deux mesures de viscosité Brookfield et Lory étant bien connues de l'homme de l'art.

En particulier, les enseignements apportés par la demande de brevet internationale WO 05/088188 sont notoirement insuffisants.

En effet, la Demanderesse a constaté qu'un produit réputé homogène, c'est-à-dire transformé de façon uniforme par réticulation en une seule phase, qu'elle soit humide, semi-sèche ou sèche, ne permettait pas d'accéder à des compositions permettant de répondre à l'ensemble des exigences et contraintes, notamment aux cisaillements intenses subis sur les machines modernes et au recyclage des colles.

En outre, cette demande internationale ne revendique, pour la pratique de la réticulation homogène, que l'exploitation de conditions dites semi-sèches, jugées trop restrictives par la Demanderesse.

Seule, la demande de brevet européenne EP 0 450 729 s'est orientée vers des mélanges d'amidons et traite d'une composition adhésive intéressant le carton ondulé et comportant, en partie primaire, un mélange d'amidon natif et d'amidon réticulé.

De nombreux agents de réticulation y sont cités, dont le trimétaphosphate de sodium.

Mais, outre le fait que ledit trimétaphosphate de sodium n'est, en aucune façon, mis en exergue dans cette demande, on constate à la lecture de cette demande que, tout comme dans le brevet US 4 339 331 cité précédemment, les inventeurs s'abstiennent de toute précision quant à la pratique même de la réticulation, laissant ainsi entendre qu'ils s'appuient sur les pratiques répandues, notamment de températures relativement élevées telles qu'elles sont exposées, de façon courante, dans la littérature. Dans le cadre d'un propos manquant ainsi de précision quant aux agents de réticulation et à leur utilisation, aucune revendication ne les concerne.

En tout état de cause, la Société Demanderesse considère que les mélanges contenant au moins un amidon non réticulé en tant que composant de la partie primaire, comme il est revendiqué dans ladite demande de brevet européen, ne permettent pas de répondre au problème tel qu'il est posé à partir des conditions de fonctionnement des matériels modernes.

En effet, à l'inverse des enseignements présentés dans celle-ci, la Demanderesse a pu observer et établir que de telles compositions, élaborées selon ce document, ne permettaient pas d'offrir toutes les garanties nécessaires, plus particulièrement en termes de rhéologie, c'est-à-dire, tant du point de vue de leur texture que de leur viscosité, notamment en ce qui concerne la stabilité de celle-ci, ainsi que la résistance qu'elle présente aux cisaillements intenses exercés sur lesdits matériels modernes, par exemple, lors du stockage ou du recyclage des préparations.

Il existe ainsi un réel besoin à concilier les exigences en termes :
- de rhéologie, notamment, de niveau de viscosité, de texture, de stabilité, tant au repos que dans les conditions de re-circulation, notamment d'absence de zone sujette à un épaississement exagéré, de comportement adapté au cisaillement, d'absence de projections sur machine, même rapide,
- de caractère filmogène,
- d'adhésivité, notion globale regroupant celles relatives à l'adhésion aux matériaux à coller, à l'adhésivité initiale ou dite « à vert », à la maturation du joint de collage, à l'efficacité du collage à sec, à sa résistance à l'état humide ou à l'eau, tout
- en satisfaisant aux critères d'accès à des amidons suffisamment peu coûteux,
- en permettant une préparation aisée de la composition et,
- en répondant, de préférence, aux impératifs liés au contact alimentaire.

Il est ainsi du mérite de la Demanderesse d'avoir pu déterminer qu'une composition caractérisée en ce qu'elle contient un mélange d'au moins un amidon de blé ayant subi une opération de réticulation moyenne ou forte pratiquée à une température inférieure à 40 °C avec les seuls trimétaphosphates, notamment avec le trimétaphosphate de sodium (TMPNa), pouvait prétendre satisfaire l'homme de l'art en conciliant l'ensemble de ces exigences, y compris le respect des règles en usage pour le contact avec les aliments.

Plus précisément, ladite composition adhésive aqueuse est caractérisée en ce qu'elle comprend un mélange comprenant au moins un amidon de blé moyennement ou fortement réticulé par l'action d'un ou de plusieurs trimétaphosphates, notamment par l'action d'un trimétaphosphate de sodium, ayant une teneur en phosphore, exprimée en P₂O₅, comprise entre 69 et 70%, présent(s) en quantité, calculée par rapport à l'amidon sec, supérieure à 0,2%, durant un temps nécessaire à un rendement suffisant de la réaction, en particulier au moins égal à 2 heures, en phase lait, c'est-à-dire sur l'amidon granulaire dispersé dans l'eau à une concentration comprise entre 30 et 45 % de matières sèches, et à une température inférieure à 40°C, de préférence à une température comprise entre 5 et 35°C, de préférence encore, à une température comprise entre 10 et 30°C, et au moins un amidon faiblement réticulé, de préférence, par l'action d'un ou de plusieurs trimétaphosphates, notamment par l'action du trimétaphosphate de sodium considéré, ayant une teneur en phosphore, exprimée en P₂O₅, comprise entre 69 et 70%, présent(s) en quantité, calculée par rapport à l'amidon sec, inférieure ou égale à 0,2% et à une température inférieure à 40°C, le ratio de l'amidon moyennement ou fortement réticulé à l'amidon faiblement réticulé étant compris entre 99/1 et 1/99, de préférence, entre 95/5 et 5/95.

A l'issue de travaux approfondis ayant pour but de déterminer et de choisir, dans le cadre desdits mélanges, les méthodes de réticulation les plus adaptées, la Demanderesse a pu observer que la réticulation de l'amidon à l'état granulaire en phase lait, c'est-à-dire sous forme d'une dispersion présentant une matière sèche comprise entre 30 et 45%, de préférence, de l'ordre de 40%, était tout à fait adaptée.

Le fait est d'autant plus marqué que la réticulation est plus forte.

Ainsi, la Demanderesse considère que, dans le cadre de l'invention, un moyen privilégié de préparation de l'amidon de blé moyennement ou fortement réticulé consiste en un procédé de réticulation dudit amidon de blé, de préférence, en phase lait, c'est-à-dire à une teneur en matière sèche comprise entre 30 et 45%, par l'action d'au moins 0,2% d'un ou de plusieurs trimétaphosphates, comptés en trimétaphosphate par rapport à l'amidon sec, notamment par l'action du trimétaphosphate de sodium, à une température inférieure à 40°C, de préférence à une température comprise entre 5 et 35°C, de préférence encore, à une température comprise entre 10 et 30°C.

La réticulation en phase sèche est, elle aussi, apparue particulièrement avantageuse quand elle est pratiquée dans le cadre d'une méthode dite séquencée, sur tout mélangeur continu adapté, en particulier équipé de dispositifs permettant de faire varier et de changer aisément les dosages, notamment celui de l'agent de réticulation, à tout moment souhaité.

Par « méthode dite séquencée », on entend la mise en place, à partir d'une même fourniture d'amidon, de préférence, de périodes (séquences) de réticulation d'une durée suffisante et en continu, à au moins deux niveaux de réticulation bien distincts, permettant de considérer que deux produits réticulés au moins, conformes à l'invention, sont alternativement obtenus.

Ces produits, présentant des niveaux de réticulation différents, sont de préférence mélangés en ligne de façon à obtenir le mélange selon l'invention.

La Demanderesse considère ainsi qu'une méthode particulièrement avantageuse de préparation du mélange selon l'invention, méthode dite séquencée, comprend différentes périodes (séquences) de réticulations bien distinctes et successives, telles que :
- la réticulation, à un dosage supérieur à 0,2% de trimétaphosphate de sodium du commerce ayant une teneur en phosphore, exprimée en P₂O₅, comprise entre 69 et 70%, présent(s) en quantité, calculée par rapport à l'amidon sec, durant un temps t1, compris entre 1 secondes et 1 heure, de préférence, compris entre 30 secondes et 10 minutes,
- la réticulation, à un dosage inférieur à 0,2% de trimétaphosphate de sodium du commerce ayant une teneur en phosphore, exprimée en P₂O₅, comprise entre 69 et 70%, présent(s) en quantité, calculée par rapport à l'amidon sec, durant un temps t2, compris entre 1 secondes et 1 heure, de préférence, compris entre 30 secondes et 10 minutes.

Les temps de réticulation respectifs, t1 et t2, peuvent être réglés, par exemple, à débit constant, pour le respect des ratios souhaités.

Une telle méthode autorise, avantageusement, la multiplication des niveaux de réticulation, dès lors qu'ils sont mis en place dans l'esprit de l'invention.

Une autre variante peut consister en différents points d'introduction d'amidons de sources diverses, dès lors que l'amidon le plus réticulé est un amidon de blé.

De préférence, la composition est caractérisée en ce que le ratio R de la partie d'amidon(s) moyennement ou fortement réticulé(s) à la partie d'amidon(s) faiblement réticulé(s) est compris entre 80/20 et 10/90, de préférence, compris entre 60/40 et 30/70.

Selon une variante, l'amidon moyennement ou fortement réticulé subit avant, pendant ou après l'opération de réticulation, au moins une modification autre que la réticulation, choisie parmi les modifications chimiques, thermochimiques, thermiques et/ou thermomécaniques. La composition selon l'invention est, de préférence, caractérisée en ce que l'amidon faiblement réticulé est choisi parmi les amidons, notamment les amidons de céréales et, en particulier, de blé, natifs.

Selon une autre variante, l'amidon (plus) faiblement réticulé subit avant, pendant ou après l'opération de réticulation, au moins une modification autre que la réticulation, choisie parmi les modifications chimiques, thermochimiques, thermiques et/ou thermomécaniques.

Plus précisément, la composition selon l'invention est caractérisée en ce que l'amidon (plus) faiblement réticulé est un amidon modifié obtenu par au moins une modification choisie dans le groupe constitué par l'hydrolyse enzymatique ou acide, les modifications thermiques ou thermomécaniques telles que, par exemple, la prégélatinisation ou l'extrusion, les modifications chimiques, notamment l'oxydation, l'estérification et l'éthérification ou encore, thermochimiques, notamment la dextrinification.

D'autres modifications physiques sont encore possibles, telles que, par exemple, les opérations thermiques connues sous les appellations de « Hot Moisture Treatment » ou d'« Annealing ».

La composition adhésive selon l'invention, élaborée selon les règles émises, permet d'accéder à un procédé amélioré de préparation de carton ondulé, caractérisé lui-même en ce qu'il comprend, au moins une fois, les étapes suivantes de distribution, sur les sommets des cannelures d'une bande de papier préformée, d'une composition adhésive selon l'invention, d'application d'un papier ou d'un carton plat sur les sommets de cannelures ainsi revêtus de la composition adhésive selon l'invention, et d'un séchage.

Selon de multiples variantes possibles, la composition adhésive aqueuse selon l'invention peut, par exemple, associer à l'amidon de blé réticulé à basse température, d'autres polymères solubles, tels que, par exemple, les poly(alcool de vinyle), les dérivés de cellulose, tels les carboxyméthylcelluloses ou les méthylcelluloses, les alginates et diverses gommes.

Elle peut aussi contenir des polymères en dispersion aqueuse, notamment des latex de toutes natures.

D'autres variantes possibles trouvent leur origine dans la nécessité de prendre en compte des considérations supplémentaires souvent imposées à l'homme de l'art. Il peut être ainsi contraint d'apporter à la composition des agents fonctionnels complémentaires, comme des lubrifiants, des azurants optiques, des plastifiants, des colorants, notamment solubles, des adoucissants, des résines hydrophobes, des agents de réticulation spécifiques de polymères autres que l'amidon, ou encore des charges ou des pigments minéraux, comme le carbonate de calcium et les kaolins, ou des charges et pigments organiques, notamment d'origine végétale comme certaines celluloses.

Le plus généralement, un soin particulier est apporté à l'adjonction d'agents plastifiants efficaces, notamment choisis parmi l'urée et ses dérivés, et les polyols, tels que, notamment, le sorbitol ou les sirops de glucose hydrogénés.

De façon plus générale, l'amidon de blé moyennement à fortement réticulé, obtenu par une opération de réticulation avec un ou plusieurs trimétaphosphates, notamment avec le trimétaphosphate de sodium (TMPNa), à une température inférieure à 40°C, de préférence, comprise entre' 5 et 35°C, de préférence encore, à une température comprise entre 10 et 30°C, est apte à être utilisé dans divers domaines industriels tels que, par exemple, ceux relatifs à l'industrie pharmaceutique, de la cosmétique et des produits d'hygiène, des industries alimentaire, papetière, textile, du cuir, des matériaux de construction ou de l'industrie pétrolière.

De même, le produit de mélange comprenant au moins un amidon de blé moyennement à fortement réticulé selon l'invention, et au moins un amidon faiblement réticulé, à une température inférieure à 40°C, est apte à être utilisé dans divers domaines industriels tels que, par exemple, ceux relatifs à l'industrie pharmaceutique, de la cosmétique et des produits d'hygiène, des industries alimentaire, papetière, textile, du cuir, des matériaux de construction ou de l'industrie pétrolière.

Les différents aspects de la présente invention, relatifs à la formulation et à l'élaboration de compositions adhésives usuelles, vont être décrits de façon plus détaillée à l'aide des exemples qui suivent, qui ne sont aucunement limitatifs.

### Exemple 1 :

Cet exemple illustre l'obtention d'un amidon de blé moyennement réticulé avec un trimétaphosphate de sodium du commerce (dénommé par la suite, TMPNa) à 40°C, ledit trimétaphosphate du commerce répondant aux critères chimiques suivants :
- teneur en phosphore, exprimée en P₂O_{5 :} comprise entre 69 et 70%,
- pH à 1% : compris entre 6 et 9,
- arsenic : 1 ppm maxi,
- métaux lourds: 10 ppm maxi,
- sulfate : 1 % maxi,
- cuivre : 10 ppm maxi,
- fluor : 10 ppm maxi,
- matières insolubles: 0,1 % maxi.

Dans un premier temps, il s'agit d'évaluer les caractéristiques des compositions adhésives aqueuses confectionnées à partir dudit amidon de blé moyennement réticulé en évaluant leur aptitude à être utilisées pour le collage de cartons ondulés.

Dans ce but, diverses opérations de réticulation de l'amidon de blé, considérée comme moyenne, sont conduites à cette température de 40°C :
en utilisant des quantités de TMPNa, variant entre 0,2% et 0,6%, comptées en TMPNa par rapport à l'amidon de blé sec, et
en appliquant des temps de réaction compris entre 2 et 10 heures.

Dans tous les cas envisagés de préparation de produits, la confection de compositions ou colles, après adaptation des formules, est possible et conduit à des compositions présentant des comportements rhéologiques satisfaisants.

Les compositions dignes d'intérêt, et notamment celles issues d'un amidon de blé réticulé avec 0,4% de TMPNa, durant 6 heures à 40°C (ABTMP4-6), sont étudiées et comparées à d'autres compositions selon l'art antérieur, l'une élaborée à partir d'amidon de maïs en partie primaire, et de fécule de pomme de terre en partie secondaire (AA1MF), l'autre, à partir d'amidon de blé réticulé à l'urée-formol (AA2ABUF).

Les formules de type Stein-Hall et les préparations correspondantes sont les suivantes :

| | AA1MF | AA2ABUF (reticulaton urée-formol) | ABTMP4-6 (reticulation TMPNa) |
|---|---|---|---|
| Partie primaire | | | |
| Eau | 370 | 450 | 450 |
| Amidon primaire | 42 | 33,5 | 33,5 |
| Température Soude comptée en sec | 32°C | 30°C | 30°C |
| Temps d'agitation à 1500 tours/minute | 4,6 | 4,8 | 4,4 |
| | 11 min. | 20 min. | 20 min. |

| Partie secondaire | | | |
|---|---|---|---|
| Eau | 665 | 525 | 525 |
| Borax | 2,1 | 3 | 3 |
| Amidon secondaire | 407,5 | 387,5 | 387,5 |
| Temps d'agitation à 1500 tours/minute | | 6 min. | 6 min. |
| Soude | | 1,9 | 1,9 |
| Eau de dilution | ' | 50 | 50 |
| Incorporation à 1500trs/min | | 5 min. | 5 min. |
| Borax | 3,4 | 3,4 | 3,4 |
| Temps d'agitation à 1500 tours/minute | 12 min. | 10 min. | 10 min. |
| Total matières commerciales / colle | 30,3% | 29,1% | 29,3% |

Ces préparations font, dans un premier temps, l'objet de mesures plus précises, de viscosité, de solubilité et de point de gélatinisation.

| | AA1MF | AA2ABUF | ABTMP4-6 |
|---|---|---|---|
| Viscosité Lory | 31,5 sec. | 35 sec. | 36 sec. |
| Viscosité Brookfield | 600mPa.s | 3125mPa.s | 3275mPa.s |
| Indice de réfraction | 4,6 | 4,7 | 4,6 |
| Point de gélatinisation | 49,5°C | 49,5°C | 49°C |

La formulation AA1MF révèle une viscosité Brookfield trop faible et une texture trop « longue », selon la qualification usuelle de l'homme de l'art.

Les compositions sont ensuite soumises à un test comparatif dit de collage à vert.

Pour cela, on réalise un collage entre un papier de cannelure et un papier de couverture. Le joint de colle formé est soumis à un chauffage à 75°C durant un temps établi comme une variable.

Après ce temps de chauffe, le joint de colle est soumis à un effort immédiat de séparation (temps fermé CT=0) des deux papiers. On mesure la force qu'il est nécessaire d'appliquer pour y parvenir.

On effectue le nombre de mesures suffisant pour évaluer le temps de chauffage nécessaire à l'obtention d'une résistance du joint de colle de 150mJ.

Il est possible de répéter l'opération à une différence près, correspondant à l'application d'un temps fermé CT égal à 5 secondes (CT5).

**Performances de collage :**

| Adhésivité à vert à 75°C | AA1MF | AA2ABUF | ABTMP 4-6 |
|---|---|---|---|
| CT0 (secondes) | 16,6 | 24,6 | 52,6 |
| CT5 (secondes) | 13,5 | 18,2 | 43,1 |

Si la rhéologie offerte par l'amidon de blé réticulé avec TMPNa à 40°C, dans les conditions de l'art antérieur (demande PCT WO 05/088188) est assez satisfaisante, par contre, la cinétique et le développement du collage du carton aux premiers instants ne sont pas suffisants pour satisfaire aux exigences de l'homme de l'art.

Le cadre de la réticulation homogène avec TMPNa, telle que revendiquée dans la demande PCT WO 05/088188, conduit même à un produit offrant des performances sensiblement inférieures à celles autorisées par la réticulation à l'urée-formol.

Le contact alimentaire, permis par la réticulation avec le TMPNa, n'est ainsi possible qu'au détriment de la performance.

### Exemple 2 :

Cet exemple est destiné à démontrer l'intérêt de mélanges d'un amidon de blé, présentant un degré de réticulation assez élevé à élevé, avec un amidon, notamment de blé, faiblement réticulé, face aux produits de l'art antérieur, notamment, face à un produit homogène, transformé en phase lait ou en phase sèche tel que décrit dans la demande de brevet internationale WO 05/088188.

Pour cela, sur les bases de l'exemple 1, on considère deux formulations adaptées à des préparations de type Stein-Hall, l'une, ABTMP 10-2, établie à partir d'un mélange d'une partie d'amidon de blé moyennement réticulé (0,2% de TMPNa durant 10 heures à 40°C) pour une partie d'amidon de blé faiblement réticulé (0,18% pendant 2 heures à 40°C) et l'autre, reprenant la formule ABTMP4-6 de l'exemple 1, élaborée à partir d'un seul amidon de blé, réticulé de façon uniforme, à 40°C.

| | ABTMP 10-2 (selon l'art antérieur) | ABTMP4-6 (selon l'art antérieur) |
|---|---|---|
| Partie primaire | | |
| Eau | 450 | 450 |
| Amidon primaire | 45 | 33,5 |
| Température | 30°C | 30°C |
| Soude comptée en sec | 6 | 4,4 |
| Temps d'agitation à 1500 tours/minute | 20 minutes | 20 minutes |
| Partie secondaire | | |
| Eau | 525 | 525 |
| Borax | 3 | 3 |
| Amidon secondaire | 380 | 387,5 |
| Temps d'agitation à 1500 tours/minute | 6 minutes | 6 minutes |
| Soude comptée en sec | 1,9 | 1,9 |
| Eau de dilution | 50 | 50 |
| Incorporation | 5 minutes | 5 minutes |
| Borax | 3,4 | 3,4 |
| Temps d'agitation à 1500 tours/minute | 10 minutes | 10 minutes |
| Total matières commerciales/colle | 29,3% | 29,3% |

Les conditions d'élaboration des formules conduisent à des compositions dignes d'intérêt qui sont assez voisines.

| | ABTMP 10-2 (selon l'art antérieur) | ABTMP 4-6 (selon l'art antérieur) |
|---|---|---|
| Viscosité Lory | 38 sec. | 36 sec. |
| Viscosité Brookfield | 2475 mPa.s | 3275 mPa.s |
| Indice de réfraction | 5,0 | 4,6 |
| Point de gélatinisation | 50,5°C | 49°C |

Les caractéristiques rhéologiques des deux formulations, pourtant relativement similaires, sont différentes : le mélange ABTMP10-2 permet d'accéder à une colle d'une texture tout à fait adaptée à l'application, à l'inverse du produit ABTMP4-6 qui conduit à une texture exagérément courte.

La formulation à partir de ABTMP10-2 présente en outre un taux de matières solubles et un point de gélatinisation plus élevés.

**Performances de collage :**

| Adhésivité à vert à 75°C | ABTMP 10-2 (selon l'art antérieur) | ABTMP 4-6 (selon l'art antérieur) |
|---|---|---|
| CT0 (secondes) | 33,4 | 52,6 |
| CT5 (secondes) | 23,4 | 43,1 |

Si le développement du collage aux premiers instants n'est pas suffisant dans les deux cas, il est indéniable que l'approche par mélange apporte de meilleurs résultats de ce point de vue.

### Exemple 3 :

Cet exemple est destiné à démontrer l'intérêt de la pratique de la réticulation à la plus basse température, compatible avec la réalité industrielle.

Dans ce but sont comparées, selon des critères identiques à ceux de l'exemple 2, deux formulations :
La première, ABTMP 25, est élaborée par mélange d'une partie d'amidon de blé fortement réticulé à 25°C (0,2% de TMPNa pendant 10 heures), pour 2 parties d'amidon faiblement réticulé à cette même température (0,18% de TMPNa pendant 2 heures).
La seconde, ABTMP 40, ne diffère que par la température de réticulation, c'est-à-dire, 40°C au lieu de 25°C, appliquée lors de la réticulation de ses constituants.

Les formulations de type Stein-Hall sont établies comme suit :

| | ABTMP 25 (selon l'invention) | ABTMP 40 (selon l'art antérieur) |
|---|---|---|
| Partie primaire | | |
| Eau | 450 | 450 |
| Amidon primaire | 44 | 44 |
| Température | 30°C | 30°C |
| Soude comptée en sec | 4,8 | 4,8 |
| Temps d'agitation à 1500tours/minute | 20 min. | 20 min. |

| Partie secondaire | | |
|---|---|---|
| Eau | 525 | 525 |
| Borax | 3 | 3 |
| Amidon secondaire | 381 | 381 |
| Temps d'agitation -1500 tours/minute | 6 minutes | 6 minutes |
| Soude comptée en sec | 1,9 | 1,9 |
| Eau de dilution | 50 | 50 |
| Incorporation | 5 minutes | 5 minutes |
| Borax | 3,4 | 3,4 |
| Temps d'agitation -1500 tours/minute | 10 minutes | 10 minutes |

Les compositions ainsi élaborées présentent les caractéristiques rhéologiques essentielles suivantes :

| | ABTMP 25 (selon l'invention) | ABTMP 40 (selon l'art antérieur) |
|---|---|---|
| Viscosité Lory | 30sec. | 40sec. |
| Viscosité Brookfield | 1710mPa.s | 1800mPa.s |
| Indice de réfraction | 4,7 | 4,7 |
| Point de gélatinisation | 51,5°C | 52,5°C |

La texture de la colle est sensiblement plus courte dans le cas des réticulations opérées à 25°C.

**Performances de collage :**

| Adhésivité à vert à 75°C | ABTMP 25 (selon l'invention) | ABTMP 40 (selon l'art antérieur) |
|---|---|---|
| CT0 (secondes) | 18,3 | 24,4 |
| CT5 (secondes) | 12,4 | 16,5 |

La différence est importante : le temps de développement du collage, correspondant à une résistance de 150mJ est réduit de 25%.

En confrontant les performances établies dans les exemples 1 et 3, on note que, seule, la réticulation avec TMPNa à basse température, c'est-à-dire à 25°C de préférence, et dans le cadre des mélanges revendiqués, permet des performances comparables, de ce point de vue, aux produits réticulés à l'épichlorhydrine.

Le trimétaphosphate (TMP) offre, en outre, l'intérêt de la salubrité et de la possibilité du contact alimentaire.

L'utilisation du trimétaphosphate, dans le cadre d'une réticulation selon l'art antérieur, c'est-à-dire à une température sensiblement plus élevée, et en l'absence de mélanges adaptés, amène des résultats très sensiblement inférieurs.

Compte tenu des corrélations habituellement observées entre ces mesures et la marche industrielle, on est en droit d'espérer un gain du même ordre en termes de vitesse de la machine.

### Exemple 4 :

Il est procédé, dans cet exemple, à des mesures supplémentaires visant à appréhender la résistance au cisaillement de produits de mélanges de deux amidons réticulés à deux niveaux de réticulation différents, comparativement à un amidon réticulé homogène, transformé en une seule phase.

Un premier mélange (mélange PL) est réalisé en associant une partie d'amidon réticulé en phase lait pendant 6 heures à 25°C, avec 0,4% de TMPNa par rapport à l'amidon sec, et deux parties d'amidon réticulé en phase lait pendant 3 heures à 25°C, avec 0,18% de TMPNa par rapport à l'amidon sec.

Dans un second cas, on procède par voie de transformation continue en phase sèche, en établissant, dans un premier temps, un programme de pulvérisation séquentielle de TMPNa, c'est-à-dire en alternant, à durées de séquences égales, deux dosages dudit agent réticulant, respectivement fixés à 0,18% et 1,85% exprimé en TMPNa sur amidon sec.

Le produit résultant (mélange SPS), soigneusement homogénéisé, est considéré comme constitué, à 50% environ, d'un produit fortement réticulé, d'une part, et à 50% environ d'un produit faiblement réticulé, d'autre part.

Les deux mélanges PL et SPS sont comparés à deux produits PHL1 et PHL2, considérés comme homogènes, obtenus en une seule phase de transformation (en lait).

Les formulations ainsi conduites présentent les caractéristiques suivantes :

| | Mélange PL selon l'invention | Mélange SPS selon l'invention | Amidon PHL1 | Amidon PHL2 |
|---|---|---|---|---|
| Partie primaire | | | | |
| Eau | 580 | 580 | 580 | 580 |
| Amidon primaire | 50 | 51 | 44,7 | 44,7 |
| Température | 42°C | 42°C | 42°C | 42°C |
| Soude comptée en sec | 4,2 | 4,2 | 4,2 | 4,2 |
| Temps d'agitation à 1500tours/min. | 20 min. | 20 min. | 20 min. | 20 min. |
| Partie secondaire | | | | |
| Eau | 615 | 615 | 615 | 615 |
| Borax | 3,15 | 3,15 | 3,15 | 3,15 |
| Amidon secondaire | 320 | 320 | 320 | 320 |
| Temps d'agitation à 1500 tours/minute | 15 min. | 15 min. | 15 min. | 15 min. |

Les trois amidons préparés, issus d'un mélange ou non, demandent les mêmes dosages pour parvenir à une composition adaptée.

Les préparations ainsi obtenues présentent les caractéristiques rhéologiques suivantes :

| | Mélange PL | Mélange SPS | Amidon PHL1 | Amidon PHL2 |
|---|---|---|---|---|
| Viscosité Lory (1) | 17 secondes | 16,5 secondes | 23 secondes | 31 secondes |
| Viscosité Brookfield (2) | 650 mPa.s | 840 mPa.s | 1130mPa.s | 1975mPa.s |
| Ratio B / L (2) / (1) | 38 | 51 | 49 | 64 |

Les différentes préparations sont ensuite soumises au cisaillement intense provoqué par l'action d'une turbine tournant à vitesse élevée, c'est-à-dire à 2500 tours/minute pendant 4 minutes.

De nouvelles mesures révèlent l'évolution de la viscosité. Exprimées en pourcentages par rapport aux premières enregistrées avant l'action mécanique, elles expriment, avec un pourcentage élevé, une bonne tenue au cisaillement.

La Demanderesse estime que, de ce point de vue, un résultat supérieur à 75% est garant d'une bonne stabilité de la colle sur machine ainsi qu'à son recyclage.

| | Mélange PL selon l'invention | Mélange SPS selon l'invention | Produit PHL1 | Produit PHL2 |
|---|---|---|---|---|
| Viscosité Lory (L) | 12,5 secondes | 11,5 secondes | 12 secondes | 15,5 secondes |
| Viscosité Brookfield (B) | 420 mPa.s | 480 mPa.s | 340 mPa.s | 510 mPa.s |
| Ratio B/L | 33,5 | 42 | 28 | 33 |

La « tenue au cisaillement » est ainsi estimée respectivement à:

| | | | | |
|---|---|---|---|---|
| Maintien de B/L | 88% | 82% | 57% | 51% |

Ladite « tenue au cisaillement » est considérée comme très insuffisante dans les cas d'utilisation des produits PHL1 et PHL2. Par contre, on note que les compositions issues de produits de mélanges voient leur texture beaucoup mieux préservée dans les conditions de cisaillement intense.

## Revendications

1. Composition adhésive aqueuse **caractérisée en ce qu'**elle comprend un mélange comprenant :
- au moins un amidon de blé moyennement ou fortement réticulé par l'action d'un ou de plusieurs trimétaphosphates, notamment par l'action d'un trimétaphosphate de sodium, ayant une teneur en phosphore, exprimée en P₂O₅, comprise entre 69 et 70%, présent(s) en une quantité, calculée par rapport à l'amidon sec, supérieure à 0,2%, durant un temps nécessaire à un rendement suffisant de la réaction, en particulier au moins égal à 2 heures, en phase lait, c'est-à-dire sur l'amidon granulaire, dispersé dans l'eau à une concentration comprise entre 30 et 45 % de matières sèches, à une température inférieure à 40°C, de préférence à une température comprise entre 5 et 35°C, de préférence encore, à une température comprise entre 10 et 30°C, et
- au moins un amidon faiblement réticulé, de préférence, par l'action d'un ou de plusieurs trimétaphosphates, notamment par l'action du trimétaphosphate de sodium considéré, ayant une teneur en phosphore, exprimée en P₂O₅, comprise entre 69 et 70%, présent(s) en une quantité, calculée par rapport à l'amidon sec, inférieure ou égale à 0,2%, et à une température inférieure à 40°C, et
- le ratio de l'amidon moyennement ou fortement réticulé à l'amidon faiblement réticulé étant compris entre 99/1 et 1/99, de préférence, entre 95/5 et 5/95.

2. Composition selon la revendication 1, **caractérisée en ce que** l'amidon de blé moyennement ou fortement réticulé est un amidon de blé natif ou modifié par au moins une modification, autre que la réticulation, choisie parmi les modifications chimiques, thermochimiques, thermiques et/ou thermomécaniques.

3. Composition selon l'une quelconque des revendications 1 ou 2, **caractérisée en ce que** l'amidon faiblement réticulé est choisi parmi les amidons, notamment les amidons de céréales et, en particulier, de blé, natifs ou modifiés par au moins une modification autre que la réticulation, choisie parmi les modifications chimiques, thermochimiques, thermiques et/ou thermomécaniques.

4. Composition selon l'une quelconque des revendications 1 et 2, **caractérisée en ce que** le ratio R de la partie d'amidon(s) fortement ou moyennement réticulé(s) à la partie d'amidon(s) faiblement réticulé(s) est compris entre 80/20 et 10/90, de préférence, compris entre 60/40 et 30/70.

5. Procédé de réticulation d'un amidon de blé par l'action d'au moins 0,2% d'un ou de plusieurs trimétaphosphates, comptés en trimétaphosphate par rapport à l'amidon sec, notamment par l'action du trimétaphosphate de sodium, ayant une teneur en phosphore, exprimée en P₂O₅, comprise entre 69 et 70%, à une température inférieure à 40°C, de préférence à une température comprise entre 5 et 35°C, de préférence encore, à une température comprise entre 10 et 30°C, et pendant une durée nécessaire à un rendement suffisant de la réaction, en particulier au moins égale à 2 heures, en phase lait, c'est-à-dire sur l'amidon granulaire dispersé dans l'eau à une concentration comprise entre 30 et 45 % de matières sèches.

6. Procédé de préparation de carton ondulé, **caractérisé en ce qu'**il comprend, au moins une fois, les étapes suivantes, de:
- distribution, sur les sommets des cannelures d'une bande de papier préformée, d'une composition adhésive selon l'une quelconque des revendications 1 à 4,
- application d'un papier ou d'un carton plat sur les sommets de cannelures ainsi revêtus de la composition adhésive,
- séchage.

7. Carton ondulé comprenant une composition adhésive selon les revendications 1 à 4.

8. Produit de mélange **caractérisé en ce qu'**il comprend au moins un amidon de blé moyennement ou fortement réticulé par l'action d'un ou de plusieurs trimétaphosphates, notamment par l'action d'un trimétaphosphate de sodium, ayant une teneur en phosphore, exprimée en P₂O₅, comprise entre 69 et 70%, présent(s) en une quantité, calculée par rapport à l'amidon sec, supérieure à 0,2%, durant un temps nécessaire à un rendement suffisant de la réaction, en particulier au moins égal à 2 heures, en phase lait, c'est-à-dire sur l'amidon granulaire dispersé dans l'eau à une concentration comprise entre 30 et 45 % de matières sèches, à une température inférieure à 40°C, de préférence à une température comprise entre 5 et 35°C, de préférence encore, à une température comprise entre 10 et 30°C, et
au moins un amidon faiblement réticulé, de préférence, par l'action d'un ou de plusieurs trimétaphosphates, notamment par l'action du trimétaphosphate de sodium considéré, ayant une teneur en phosphore, exprimée en P₂O₅, comprise entre 69 et 70%, présent (s) en une quantité, calculée par rapport à l'amidon sec, inférieure ou égale à 0,2%, et à une température inférieure à 40°C,
et **en ce que** le ratio de l'amidon moyennement ou fortement réticulé à l'amidon faiblement réticulé est compris entre 95/5 et 5/95.

9. Produit de mélange selon la revendication 8, **caractérisée en ce que** le ratio R de la partie d'amidon(s) moyennement ou fortement réticulé(s) à la partie d'amidon(s) faiblement réticulé(s) est compris entre 80/20 et 10/90, de préférence, compris entre 60/40 et 30/70.

10. Utilisation d'un produit de mélange d'amidons réticulés selon l'une des revendications 8 ou 9 dans divers domaines industriels tels que, par exemple, ceux relatifs à l'industrie pharmaceutique, de la cosmétique et des produits d'hygiène, des industries alimentaire, papetière, textile, du cuir, des matériaux de construction ou de l'industrie pétrolière.
